(19) Europäisches Patentamt / European Patent Office / Office européen des brevets

(11) EP 3 735 904 A1

(12) **EUROPEAN PATENT APPLICATION**

(43) Date of publication:
**11.11.2020 Bulletin 2020/46**

(21) Application number: **19305595.1**

(22) Date of filing: **09.05.2019**

(51) Int Cl.:
*A61B 5/145* (2006.01) *A61B 5/1477* (2006.01)
*A61B 5/00* (2006.01) *G01N 27/42* (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **PIERRE FABRE MEDICAMENT**
**92100 Boulogne-Billancourt (FR)**

(72) Inventors:
- **LAUR, Clément**
  **81710 Naves (FR)**
- **CARRARA, Sandro**
  **1093 La Conversion (CH)**
- **MEHMETI, Eda**
  **8010 Graz (AT)**

(74) Representative: **Regimbeau**
**20, rue de Chazelles**
**75847 Paris Cedex 17 (FR)**

(54) **DETECTING NICOTINE IN SWEAT**

(57) Sweat is detected directly on the human skin by an electro-chemical method using a device for detecting nicotine in sweat which can be included in a dermal patch.

Figure 6

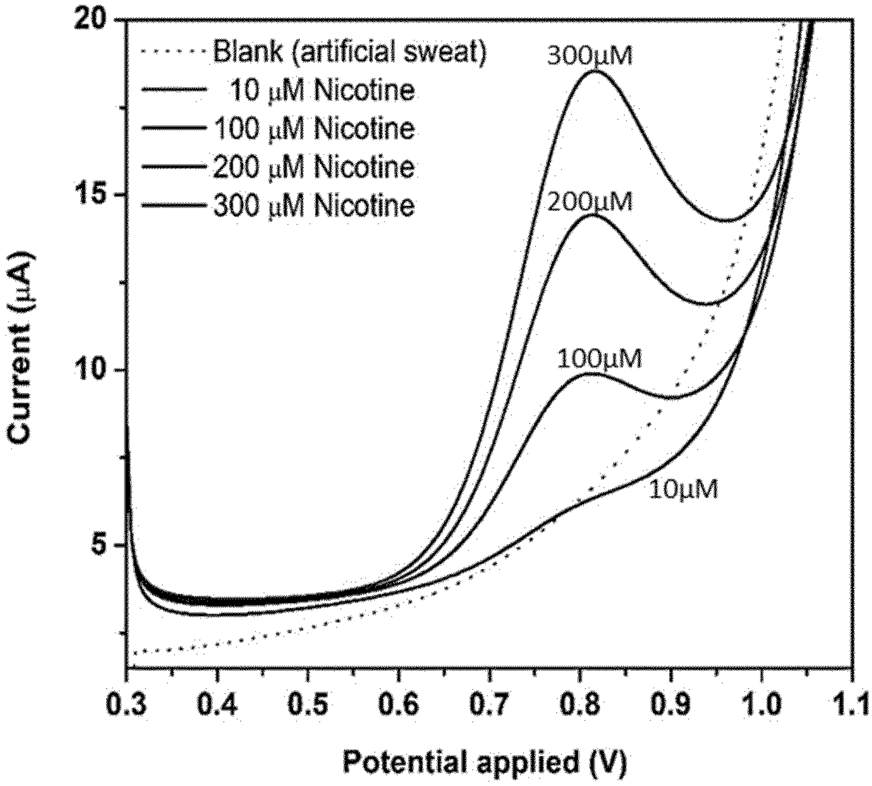


EP 3 735 904 A1

## Description

### Technical field of the invention

The present invention relates to an electrochemical method for detecting nicotine in sweat, in particular in human sweat. The present invention also relates to a device for detecting nicotine in sweat, and to a dermal patch including said device, allowing to detect nicotine in sweat directly on the human skin.

### Background of the invention

Nicotine is the main alkaloid found in tobacco leaves, accounting for about 95% of the total alkaloid content, which are used in the production of cigarettes, cigars or flake tobaccos with content varying from 1 to 30 mg/g.

Currently, more than 1.2 billion people worldwide consume different tobacco products that result in nicotine addiction. The regular intake of nicotine through smoke of the burning tobacco products is toxic for both active and passive smokers and can cause several negative outcomes in human health such as cardiovascular, respiratory, central nervous diseases and even cancer. This enormous consumption of tobacco products worldwide leads to around 4.9 million deaths per year. Therefore, several products are proposed as a replacement therapy to avoid tobacco consumption and to quit smoking. Such products include nicotine in the form of nicotine chewing gums, nicotine patches, and nicotine tablets. Absorption of nicotine can be through oral intake, lungs, urinary bladder, gastrointestinal tract, and its base form can be also easily absorbed through the skin, and can cause a poisoning in a contact with nicotine containing pesticides too (Karaconji, I.B. Arh. Hig. Rada Toksikol, 2005, 56, p.363-371). As it is evidenced by many studies, nicotine's fatal dose in adults was specified to be 60 mg/kg.

Therefore, critical monitoring and determination of nicotine is of paramount importance in the fields of medicine, toxicology and tobacco industry, and requires the development of simple, accurate, sensitive, and selective analytical methods. For this purpose, numerous methods using gas chromatography, high performance liquid chromatography, capillary electrophoresis and spectrophotometry have been developed for the quantification of nicotine in various sample matrices. However, most of these proposed analytical methods require time-consuming sample-preparation steps, expensive instrumentation, and highly skilled workers.

Electrochemical methods offer the advantages to be inexpensive, simple, robust, sensitive and having short analysis time. In addition, nicotine is an electroactive species, therefore suitable for electrochemical detection. Several electrochemical methods have been reported so far for the detection of nicotine (Švorc L. et al., Diam. Relat. Mater., 2014, 42, pp. 1-7, 2014; Highton, L. et al. Electroanalysis, 2009, 21, p.2837). However, most of these electrochemical methods are achieved on cigarette samples and thus do not allow a direct monitoring of nicotine in human fluid samples. Highton, L. et al. discloses a method of detection of nicotine in saliva using square wave voltammetry (SWV) but the method is not very sensitive with a relatively high minimal limit of detection, at 2 $\mu$M, which prevents the detection and the quantification of a lower quantity of nicotine.

There is thus a need for a sensitive, accurate, cost-effective, easy-to-use method and device for monitoring nicotine in human fluid samples, in which interference of other substances do not prevent selective detection of nicotine.

Any device that will be designed for electrochemical detection of nicotine needs to be simple, sensitive, and rapid enough since plasma half-life of nicotine in smokers has been reported to be around 40 min (Dollery et al. Br. Med. J., 1975, 4, p.313-316).

### Summary of the invention

Surprisingly, the inventors have developed for the first time a highly sensitive, simple, rapid, and low cost method for the detection and the quantification of nicotine in sweat, in particular in human sweat, based on electrochemical sensors, and a device for the same. The present invention also provides a dermal patch comprising said device for the detection of nicotine in sweat directly collected on human skin.

The present invention thus relates to an electrochemical method for detecting nicotine in sweat, the method comprising the steps of:

(a) providing a solution comprising or consisting of sweat, and
(b) achieving an electrochemical measurement of the solution resulting from step a) with an electrochemical sensor.

The present invention also relates to a device for electrochemical detection of nicotine in sweat, said device comprising an electrochemical sensor.

Finally, the present invention is also directed to a dermal patch comprising said device for electrochemical detection of nicotine in sweat.

## Definitions

As used herein, the term "sweat" is understood as a solution consisting of sweat or comprising sweat. Sweat can be collected via sweat patches from which sweat is then extracted, diluted and analyzed. Sweat can also be collected by dermal patch applied on skin and the electrochemical measurement is directly achieved on this sweat without the step of collecting/extracting via sweat patches.

As used in the present invention, the term "nicotine" refers to the compound of following formulas, which can be di-protonated, monoprotonated or deprotonated according to the pH of the medium:

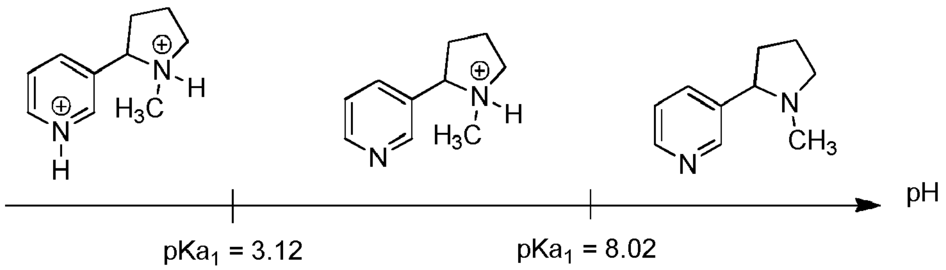


Nicotine has two pKa values: 3.12 and 8.02. In the body, nicotine can be metabolized into several compounds called metabolites. The main metabolite of nicotine is called cotinine of the following formula:

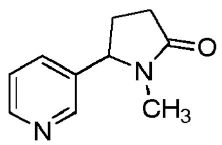

"Nicotine" may be abbreviated by "NIC" for the present invention.

Cotinine is not intended to be detect and quantified by the method of the present invention. It belongs to the interfering compounds defined below. Only nicotine is detected and quantified through the method and with the device of the present invention.

As used herein, the term "voltammetry" refers to a category of electroanalytical methods used to evaluate the oxidation behavior of an analyte. The voltammetric experiment is carried out in an electrochemical cell comprising three electrodes: a working electrode, a reference electrode and a counter electrode. In voltammetry experiment, the current flow resulting from the reduction or oxidation of compounds in solution under the effect of a variation in the potential difference between two specific electrodes, the working electrode and the reference electrode (whose potential is always fixed), is measured. The counter electrode allows the current to flow through without disturbing the value of the potential applied to the working electrode. The analytical data for a voltammetric experiment comes in the form of a voltammogram i=f(E) which plots the current produced by the analyte in function of the potential applied to the working electrode. The potential can be varied either step by step or continuously. The measured current flow is equal to the sum of the capacitive current, which is associated to the variation of the potential in the working electrode, and the faradic current, due to the oxidoreduction reaction which takes place in the electrochemical cell.

As used herein, the term "cyclic voltammetry" refers to a specific type of voltammetry in which the potential is varied, at constant speed, repeatedly between two terminals, called "inversion potentials". A "cycle" is a round trip between the two terminals. An important experimental parameter is the rate at which the potential is varied, called the scanning rate (expressed in V/s).

As used herein, the term "Differential pulse voltammetry", abbreviated DPV, refers to a type of voltammetric method in which the potential is varied using pulses of increasing amplitude and the current is sampled before and after each voltage pulse. DPV is particularly suitable when the species to be analyzed is at low concentration. Indeed, DPV enables low background currents and a low detection limit by eliminating the capacitive current. Therefore, only the faradic current is measured.

As used herein, the term "screen-printed carbon electrode" refers to Screen Printed Electrodes consisting of different layers of electrically conductive inks machine-printed on glass, plastic or ceramic, wherein at least one layer, i.e. one electrode, is made of carbon paste. Advantageously, according to the present invention, the screen-printed carbon electrode comprises a working and a counter electrode made by carbon paste (made from a mixture of conducting graphite powder and a pasting liquid), and also hosting a reference electrode made by silver and silver chloride (Ag/AgCl). In particular, the screen-printed carbon electrode used in the present invention is an unmodified screen-printed carbon

electrode, meaning that the carbon paste is not functionalized nor mixed with other substance(s).

As used herein, "sweat patches" refers to a device used for sweat diagnostic. User applies patch on skin over a period of hours or days for collecting sweat, preferably for few hours such as one or two hours. Sweat patch captures insensible perspiration, the uncontrolled loss of sweat from the skin, in the absorbent pad. An example of suitable commercially available sweat patches is PharmaCheck® Sweat Patches.

The sweat patch is typically made with a polymer film, which is a semi-permeable membrane that allows small molecules, such as oxygen, carbon dioxide, and water vapor to pass through the patch- leaving the skin underneath healthy and sterile. Substances of larger molecular weight, as nicotine, are trapped and concentrated in the absorbent pad and submitted to the laboratory for analysis. Interfering compounds present in sweat can thus also been collected. The electrochemical method for detecting nicotine according to the present invention is therefore calibrated so that the detection signal of nicotine is not affected by these interfering compounds. After collection, the patch is placed into a solvent, for example into a polar solvent such as alcohol, for extracting sweat and analyzing it using suitable electrochemical method.

As used herein, "a dermal patch" refers to a self-adhesive patch at skin temperature, placed on skin. Usually, dermal patches are used to deliver dose of drug through the skin and into the bloodstream. According to the present invention, the dermal patch is not intended to perform drug delivery but it includes a device for collecting sweat, detecting and quantifying nicotine in sweat. The dermal patch of the present invention will thus collect sweat and the included device will be able to perform the electrochemical analysis to detect and quantify nicotine in said sweat sample, directly on skin. In a dermal patch, there is no semi-permeable membrane and the water does not pass through. The sweat remains entrapped in the matrix of the dermal patch. According to this embodiment, sweat components are <u>not</u> collected from the patch nor extracted with a solvent after the patch is weared. The electrochemical measure is not achieved in a sequential way after sweat components collection and extraction, in a separate electrochemical cell. With a dermal patch including the device of the present invention, the electrochemical measurement to detect, and optionally quantify, nicotine in sweat is achieved directly in the dermal patch, simultaneously with the sweat collecting. Typically, the dermal patch according to the present invention is able to collect between 0.5mL to 50 mL of sweat over an adequate time period, in particular between 5 mL and 30 mL.

As used herein, "artificial sweat" is understood as a mixture of different salts and compounds (ammonium chloride, lactic acid, urea, acetic acid, sodium chloride, see the examples) that mimics the physico-chemical conditions of natural sweat. According to the present invention, artificial sweat can comprise different known concentrations of nicotine in order to establish a calibration curve for the electrochemical measurement.

As used herein, "interfering compounds" are understood as the electrolyte and organic compounds naturally present in sweat, in particular in human sweat. In particular, sweat includes: glucose, ascorbic acid, uric acid, pyruvic acid, dopamine, glutamic acid and ions such as $Ca^{2+}$, $Mg^{2+}$, $Zn^{2+}$ and $Pb^{2+}$. According to the present invention, metabolites of nicotine such as cotinine are also interfering compounds which can be present in smoker's sweat.

As used herein, "quantifying nicotine in sweat" means that the quantity of nicotine contained in sweat collected by the sweat patch or the dermal patch over a period of time can be determined with the method of the present invention.

According to the present invention, all potentials given in the description are versus the pseudo reference electrode at room temperature. A "pseudo reference electrode", is typically the Ag/AgCl electrode usually found in commercially available screen printed electrodes. The term "pseudo" is used because the performance of such kind of electrodes is not exactly as robust as for the best membrane-based reference electrodes usually used in electrochemistry.

## Detailed description

In a first aspect, the present invention relates to an electrochemical method for detecting nicotine in sweat, in particular human sweat, the method comprising the steps of:

(a) providing a solution comprising or consisting of sweat, and
(b) achieving an electrochemical measurement of the solution resulting from step a) with an electrochemical sensor.

The electrochemical measurement is preferably a voltammetric measurement, in particular a differential pulse voltammetric measurement. Step b) thus typically provides a voltammogram I=f(E) in which the oxidation peak of the nicotine is detected when nicotine is present in sweat.

Advantageously, the electrochemical sensor used in the method of the present invention comprises a screen-printed carbon electrode as defined in the present invention. Said screen-printed carbon electrode comprises a working electrode, preferably made of carbon, a reference electrode, preferably made of silver/silver chloride, and a counter electrode, typically made of a conductive material, for example selected from the group consisting of carbon or metals such as gold or platinium. Advantageously, the working electrode and the counter electrode are both made of carbon and the reference electrode is made of silver/silver chloride.

According to one embodiment, the method comprises an intermediate step a') between step a) and step b) of transferring the solution comprising or consisting of the sweat to the electrochemical sensor.

Typically, step a) is carried out by collecting the sweat and diluting it in a buffered electrolytic solution. Sweat can be collected by using sweat patches which are for example applied to smoker's skin. Nicotine is then extracted from the sweat patches using a solvent, in particular a polar solvent. Preferably, the solvent is an alcohol such as methanol, ethanol, propanol, butanol. More preferably, the solvent is methanol. The extract is then concentrated under reduced pressure and the residue is diluted in a buffered electrolytic solution.

According to this embodiment, the electrolytic buffered solution has a pH equal to the physiological pH, typically comprised between 5 and 8. For example, the buffered electrolytic solution is a phosphate buffer solution having a pH of 7.4. The oxidation process within the voltammetry experiment is affected by pH value. Increasing pH of the supporting electrolyte from 5 to 7.4 is associated with an increase of the oxidation peak current of nicotine, with a maximum observed at pH 7.4. At a pH lower than 5, the current responses of nicotine are not detectable. At pH values above 8, the oxidation peak potentials of nicotine shifted towards less positive values, indicating proton participation in the electrode reaction. Physiological pH of the electrolyte is the optimum pH in order to maintain a stable form of nicotine during the experiment.

According to a preferred embodiment, the electrochemical method as described above enables to quantify nicotine in a solution comprising or consisting of sweat, such as human sweat. According to this embodiment, the method comprises a further step c) of quantifying nicotine in sweat. In this purpose, a calibration curve of I=f(C) is made by using artificial sweat samples comprising different known concentrations of nicotine. The peak current is measured for each concentration via electrochemical measurement, typically a DPV measurement, in the same conditions than those used for achieving the electrochemical measurement of step b). Therefore, in order to quantify the concentration of nicotine in a random sample, the voltammogram I=f(E) resulting from step b) is compared to the calibration curve.

According to a preferred embodiment, the differential pulse voltammetric measurement performed during step b) is achieved in a potential range between -2V and + 2V, in particular between -1V and +1.5V, most preferably between 0V and +1.3 V.

Advantageously, the pulse amplitude of the differential pulse voltammetric measurement is comprised between 10 and 200 mV, preferably between 80 and 160 mV, more preferably between 100 and 150 mV. In a preferred embodiment, the pulse amplitude is 140 mV.

Advantageously, the pulse time of the differential pulse voltammetric measurement is comprised between 10 and 70 ms, preferably between 20 and 40 ms, more preferably the pulse time is 20 ms or 40 ms.

According to one embodiment, the potential scan rate of the differential pulse voltammetric measurement is comprised between 10 and 300 $mV.s^{-1}$, preferably between 20 and 200 $mV.s^{-1}$, more preferably between 30 and 100 $mV.s^{-1}$. In a preferred embodiment, the potential scan rate is 50 $mV.s^{-1}$.

According to a preferred embodiment, the differential pulse voltammetry measurement is performed in a potential range between 0V and +1.3V, with a pulse amplitude comprised between 100 and 150mV, preferably of 140 mV, with a pulse time of 20 or 40 ms, and with a potential scan rate comprised between 30 and 100 $mV.s^{-1}$, preferably of 50 $mV.s^{-1}$.

According to a particular embodiment, the electrochemical method of the present invention can detect nicotine in a sample with a minimal concentration of at least 0.10 μM, particularly of at least 0.30 μM, more particularly of at least 0.50 μM, even more particularly of at least 0.60 μM. Preferably, the electrochemical method can detect nicotine in a range of concentrations comprised between 0.10 μM and 400 μM, or between 0.30 μM and 400 μM, or between 0.50 an μM and 400 μM, or between 0.60 μM and 400 μM, or between 0.80 μM and 400 μM, or even between 1.0 μM and 400 μM.

According to another particular embodiment, the electrochemical method of the present invention can quantify nicotine in a sample with a minimal concentration of at least 1.0 μM, particularly of at least 1.50 μM, more particularly of at least 1.90 μM, even more particularly of at least 2.0 μM. Particularly, the electrochemical method can quantify nicotine in a range of concentrations comprised between 1.0 μM and 400 μM, or between 1.50 μM and 400 μM, or between 1.90 an μM and 400 μM, or between 2.0 μM and 400 μM.

Sweat comprises a multitude of other analytes whose oxidation behavior during the electrochemical measurement may interfere during the electrochemical analysis of sweat for detecting nicotine. For example, sweat comprises common ions such as $Ca^{2+}$, $Mg^{2+}$, $Zn^{2+}$, $Pb^{2+}$, organic compounds such as glucose, ascorbic acid, uric acid, pyruvic acid, dopamine, glutamic acid or metabolites of nicotine such as cotinine. The electrochemical method of the present invention is sufficiently selective for nicotine and can be achieved in presence of such interfering compounds without disrupting nicotine detection.

In a second aspect of the present invention, a device for electrochemical detection of nicotine in a solution comprising or consisting of sweat is provided, said device comprising an electrochemical sensor. In particular, the device of the present invention is for detecting nicotine in human sweat.

According to one embodiment, the electrochemical sensor is suitable to perform a voltammetric measurement, in particular a differential pulse voltammetric measurement.

In particular, the electrochemical sensor comprises a screen-printed carbon electrode as defined above. Advantageously, in said screen-printed carbon electrode, the working electrode and the counter electrode are both made of carbon and the reference electrode is made of silver/silver chloride.

In a preferred embodiment, the electrochemical sensor is suitable to be immersed or contacted in a solution comprising or consisting of sweat.

According to a particular embodiment, the solution comprising the sweat intended to be analyzed is a buffered electrolytic solution. Advantageously, said buffered electrolytic solution has a pH equal to the physiological pH, typically comprised between 5 and 8. In one example, the buffered electrolytic solution is a phosphate buffer solution having a pH of 7.4.

According to a further preferred embodiment, the device of the present invention quantifies nicotine in samples comprising or consisting of sweat.

Advantageously, the device of the present invention can detect nicotine in a sample with a minimal concentration of at least 0.10 μM, particularly of at least 0.30 μM, more particularly of at least 0.50 μM, even more particularly of at least 0.60 μM. Preferably, the electrochemical method can detect nicotine in a range of concentrations comprised between 0.10 μM and 400 μM, or between 0.30 μM and 400 μM, or between 0.50 an μM and 400 μM, or between 0.60 μM and 400 μM, or between 0.80 μM and 400 μM, or between 1.0 μM and 400 μM. In an alternative, the device of the invention can quantify nicotine in a sample with a minimal concentration of at least 1.0 μM, particularly of at least 1.50 μM, more particularly of at least 1.90 μM, even more particularly of at least 2.0 μM. Particularly, the device can quantify nicotine in a range of concentrations comprised between 1.0 μM and 400 μM, or between 1.50 μM and 400 μM, or between 1.90 an μM and 400 μM, or between 2.0 μM and 400 μM.

Preferably, conditions for differential pulse voltammetric measurement achieved by the device of the present invention are the following:

- the potential range is between -2V and + 2V, in particular between -1V and +1.5V, most preferably between 0V and +1.3 V.
- the pulse amplitude is comprised between 10 and 200 mV, preferably between 80 and 160 mV, more preferably between 100 and 150 mV; in a preferred embodiment, the pulse amplitude is 140 mV,
- the pulse time is comprised between 10 and 70 ms, preferably between 20 and 40 ms, more preferably the pulse time is 20ms or 40 ms.
- the potential scan rate is between 10 and 300 $mV.s^{-1}$, preferably between 20 and 200 $mV.s^{-1}$, more preferably between 30 and 100 $mV.s^{-1}$; in a preferred embodiment, the potential scan rate is 50 $mV.s^{-1}$.

According to a preferred embodiment, the device of the present invention achieved a differential pulse voltammetry measurement in a potential range between 0V and +1.3V, with a pulse amplitude comprised between 100 and 150mV, preferably of 140 mV, with a pulse time of 20 or 40 ms, and with a potential scan rate comprised between 30 and 100 $mV.s^{-1}$, preferably of 50 $mV.s^{-1}$.

According to a preferred embodiment, the device of the present invention is included in a dermal patch. Preferably, the dermal patch is a self-adhesive dermal patch.

According to one embodiment, the dermal patch comprising the device of the present invention is applied on human skin, anywhere on the body. In a particular embodiment, the dermal patch is applied on shoulders.

According to this embodiment, sweat is not collected with sweat patches from which sweat is then extracted. The device of the present invention included in the dermal patch achieves the electrochemical measurement directly on perspiration on the skin, in particular on insensible perspiration.

The present invention also relates to the use of the device as described above for electrochemical detection of nicotine in sweat, in particular via a voltammetric measurement such as differential pulse voltammetric measurement.

In particular, the use of the present invention enables to detect nicotine in sweat, advantageously at concentration of at least 0.10 μM, preferably at least 0.30 μM, more preferably of at least 0.50 μM, even more preferably of at least 0.60 μM.

Advantageously, the present invention relates to the use of the device as described above for quantifying nicotine in samples comprising or consisting of sweat.

In yet another aspect, the present invention relates to a method for detecting nicotine in sweat by using the device as described above. According to another embodiment, the present invention relates to a method for quantifying nicotine in sweat by using the device of the present invention.

A dermal patch comprising a device as described above for detection of nicotine in sweat, in particular in human sweat, is also provided according to the present invention.

**Description of the Figures**

**Figure 1.** Cyclic voltammograms obtained at the SPCE in the absence (Blank; dashed-line,) and presence of 1 mM nicotine (solid-line) in 0.1 M PBS (pH 7.4) at a scan rate of 100 mV $s^{-1}$.

**Figure 2.** Effect of the pH value on the peak potential (○) and peak current (□) of 1 mM nicotine at the SPCE in 0.1 M PBS (pH 5.0 - 8.0) using cyclic voltammetry at a scan rate of 100 mV $s^{-1}$.

**Figure 3. A)** Cyclic voltammograms obtained at the SPCE in the absence (Blank; dashed-line) and presence of 1 mM nicotine in 0.1 M PBS (pH 7.4) at different scan rates from 10 to 300 mV $s^{-1}$. **B)** Ip as a function of $v^{1/2}$ for the oxidation peak of NIC.

**Figure 4. A)** Differential pulse voltammograms obtained at the unmodified SPCE in the absence (Blank; dashed-line) and presence of different concentrations of nicotine (1-375 μM) in 0.1 M PBS (pH 7.4) at pulse amplitude of 140 mV, pulse time 20 ms and scan rate of 50 mV $s^{-1}$. **B)** Ip as a function of NIC concentration including the error bars.

**Figure 5.** Signals of tested interfering compounds in the same concentration with nicotine (20 μM) expressed as relative signals of nicotine at the SPCE in 0.1 M PBS (pH 7.4) at pulse amplitude of 140 mV, pulse time 20 ms and scan rate of 50 mV $s^{-1}$.

**Figure 6.** Differential pulse voltammograms obtained at the SPCE showing the analysis of the artificial sweat samples in absence (Blank; dashed-line) and in presence of NIC at 10, 100, 200 and 300 μM (first, second, third and fourth solid-lines from bottom to top) at pulse amplitude of 140 mV, pulse time 20 ms and scan rate of 50 mV $s^{-1}$.

The present invention is described in detail in the following examples, without being limited to it.

## Examples

### *Example 1: Protocol for sweat collection*

Sweat collecting patches, as a validated method for sweat collection, have been used to monitor drug abuse via chromatographic methods after extraction of sweat from patients or volunteers (Kintz et al., J. Chromatogr. B Biomed. Appl., 1998, 705, p.357-361). Those patches are composed of an absorbent pad where the sweat is concentrated, an acrylate adhesive layer for making the patch wearable on skin, and a thin transparent polyurethane film to isolate the patch from the environment. Hence, these patches are suitable also for nicotine detection. The semi permeable membrane of the patch allows water to pass through and to retain/concentrate the components of the sweat.

Commercially available PharmCheck® Sweat Patches were received from the PharmChem, Inc. consisting of a special absorbent pad where the sweat is collected. Sweat patches were applied to two apparently smoker volunteers on the measurement day, labelled as heavy (30 cigarettes/day) and light smoker (10 cigarettes/day). Sweat patches were applied according to manufacturer's protocol. Briefly, the volunteer's upper arm area, was cleaned with alcohol wipes before patch application. The volunteers performed strong physical exercises for about 1 h. Then the patches were removed, placed in a 50 mL tube and analyzed immediately on the same day. Therefore, nicotine was extracted from the whole patch by adding 5 mL of methanol to the 50 mL tube, followed by shaking for 30 min. Then, the methanol extract was removed quantitatively and evaporated to dryness. The residue was dissolved in 2 mL of PBS 0.1 M (pH 7.4) and transferred to an electrochemical cell for the measurements. Afterwards, recovery experiments were done by spiking nicotine in different concentrations. All experiments were performed three times, figure of merits for the sensing performance estimated from calibration curve, and given as mean value.

### *Example 2: Electrochemical behavior of nicotine on screen-printed carbon electrode*

Cyclic voltammetry was employed to study electrochemical behavior of NIC on SPCE. All necessary factors, which may influence to the current response of NIC were carefully studied to determine the best conditions at which the best analytical performance can be achieved.

### Instrumentation

Cyclic voltammetric and differential pulse voltammetric measurements were performed using a potentiostat/galvanostat (Autolab PGSTAT101) controlled by a computer with the corresponding software (Nova 1.10.5). The electrochemical cell was equipped with screen-printed carbon electrode. Working (4 mm diameter) and counter electrodes were made of carbon and reference electrode was made of silver/silver chloride. All of the pH values were measured

using a pH meter (model pHenomenal pH 1000 L, VWR) with a combined electrode (glass-reference electrode) which was calibrated daily using standard buffer solutions. All potentials given in the text are versus the pseudo reference electrode at room temperature.

**Measurement procedures**

Cyclic voltammetry (CV) with a scan rate of 100 mV $s^{-1}$ was used for characterizing the electrochemical behavior of nicotine at the electrode surface. The investigated solutions were transferred into the voltammetric cell and the voltammograms (usually 5 cycles) were recorded in a potential range between 0 V and +1.3 V after addition of particular concentrations of nicotine.

Figure 1 shows the corresponding voltammograms in the absence (Blank, PBS, pH 7.4) and presence of 1 mM NIC in PBS (pH 7.4) on SPCE.

During anodic scan, NIC undergoes oxidation at the surface of the SPCE starting at a potential of around +0.6 V, and showing high oxidation peak currents compared to the blank when no NIC was added. During a cathodic scan, by reversing at +1.3 V, no voltammetric response which may correspond to the reduction of NIC was observed. Thus, it may be concluded that the oxidation of NIC is irreversible at SPCE in agreement with previously evidenced and reported data. Additionally, the NIC oxidation potentials in our study, at SPCE are much lower compared to boron-doped diamond electrodes (+1.3 V and +1.45 V), suggesting a faster electron transfer rate. The obtained background current was satisfactorily low showing the benefits of the SPCE as a sensing platform.

**Effect of pH of supporting electrolyte**

Nicotine's two pKa values of 3.12 and 8.02 correspond to the mono-protonated and deprotonated form present in the molecule depending on the pH of the media. In order to optimize the pH value of the supporting electrolyte for the electrochemical oxidation of NIC at the SPCE, the effect of pH on the peak current (Ip) and the peak potential (Ep) was investigated using CV in the pH range from 5.0 to 8.0 with 0.5 pH unit increments; and additionally, the pH value of 7.4, which matches the physiological pH, was tested. When pH values were adjusted to lower than 5.0, the current responses of NIC were not detectable (data not shown). CVs were recorded in 0.1 M PBS in the presence of 1 mM NIC and the results from the plot of Ep and Ip values as a function of pH are illustrated in Fig.2. The electrochemical behavior of NIC at SPCE over the tested pH range yielded an irreversible oxidation process. The obtained results indicate an increase of the oxidation peak current of NIC when increasing the pH value of the supporting electrolyte with a maximum observed at pH 7.4 showing that the oxidation process is affected by pH value. At pH values above 7.4 oxidation peak was split in two overlapping peaks. However, the oxidation peak potentials of NIC shifted towards less positive values with the increase of the pH values, indicating proton participation in the electrode reaction. Ep is linearly pH dependent in the range from 5.0 to 7.4 and can be expressed with Eq.1:

$$Ep\ (V) = -0.073 \times pH + 1.368\ (R^2 = 0.999) \qquad (1)$$

The slope value indicates that the number of electrons and protons involved in the electrode reaction is equal. Therefore, the pH 7.4 was selected based on our results as the most suitable and optimum pH value in order to maintain a stable nicotine form during the quantification.

**Effect of potential scan rate**

In order to evaluate the nature of the electrochemical reaction on SPCE, the influence of the potential scan rate (v) on the oxidation peak current and peak potential of NIC was examined in the range from 10 to 300 mV $s^{-1}$ using CV in PBS at pH 7.4 (Fig.3A). With increase of a scan rate, the peak potential for the oxidation of NIC slightly shifted to more positive values without any cathodic peak current, confirming an irreversible electrochemical reaction. The peak current (Ip) of NIC increased practically linearly with square root of the scan rate ($v^{1/2}$) suggesting that the oxidation process on the electrode is controlled by diffusion rather than adsorption (Fig.3B). The linear dependence can be expressed by Eq.2:

$$Ip\ (\mu A) = 1.128 \times v^{1/2} + 6.438\ (R^2 = 0.998) \qquad (2)$$

***Example 3: Analytical performance of the device of the present invention***

• ***Optimization of DPV parameters***

DPV was then selected for the quantification of NIC which enables the low background currents and low detection limits.

*Measurement procedures*

DPV parameters were optimized using a concentration of 1 mM nicotine in the measurement solution in a potential range between 0 V and +1.3 V and the method was applied for further quantification of nicotine (pulse amplitude of 0.14 V and pulse time 0.04 s).

The calibration curves were evaluated from the peak currents of analyte representing the average value from three successive additions of standard solutions of nicotine.

The most important experimental parameters of the method (pulse amplitude and pulse time) were optimized since they may effect on the analytical response of the analyte. Experiments were carried out in PBS (pH 7.4) in presence of 1 mM NIC in the way that when one investigated parameter was varied the other was kept in a fixed value. Pulse amplitude was tested in the range from 10 to 200 mV (with the pulse time fixed at 25 ms). When amplitude was increasing the oxidation peak current of NIC was also increasing with concomitant widening of the peaks. Thus, pulse amplitude of 140 mV was selected as a compromised most appropriate value, with respect to the current response and peak shape of NIC. Moreover, several experiments done by keeping the pulse amplitude fixed at 140 mV but varying the pulse time in the range from 10 to 70 ms returned the value of 20 ms as the optimum, with the highest peak current and stable peak shape. Therefore, these optimized experimental parameters were employed in all next experiments.

• ***Analytical performance***

By using the optimized experimental parameters as described above, DPV was employed as electrochemical technique to study the analytical performance of the device of the invention. Differential pulse voltammograms were recorded at different NIC concentrations on the SPCE in PBS (pH 7.4). The typical voltammograms are illustrated in Fig.4A obtained by the successive additions of NIC from 1 to 375 μM and it can be seen that an increase in NIC concentration is accompanied by an increase in oxidation peak current. The relationship between the peak currents and the concentrations of NIC is shown in the Fig.4B. All data are the mean value of three measurements. The SPCE exhibits excellent linear concentration range from 1 to 375 μM for NIC. The numerical expression for the regression line in the calibration curve is given by Eq.3:

$$Ip\ (\mu A) = 0.0316\ (\pm 0.0002) \times c\ (\mu M) - 0.0795\ (\pm 0.0205)\ (R^2 = 0.999) \qquad (3)$$

The limit of detection (LOD, 3σ-value) and limit of quantification (LOQ, 10σ-value) were estimated from the calibration curve to be **0.59 μM** and **1.97 μM** NIC, respectively, which enables the detection of low concentrations of nicotine in sweat samples at physiological pH, and even lower thanks to the good fidelity of the model. The device of the present invention therefore shows significantly lower limits of detection without any need for a pre-treatment step than those disclosed in the prior art.

***Example 4: Interference studies***

**Artificial sweat solutions**

Artificial sweat solution was prepared according to the following steps. Briefly, 327 mmol/L ammonium chloride, 166 mM lactic acid, 83 mM urea, 42 mM acetic acid, 34 mM sodium chloride were dissolved in deionized water and adjusting the pH to 7.4 by addition of 2 M sodium hydroxide. All chemicals are used without any further purification.

**Interference studies**

Oxidation behavior of the most possible interfering compounds which may occur in sweat samples was analyzed including common ions: $Ca^{2+}$, $Mg^{2+}$, $Zn^{2+}$, $Pb^{2+}$; organic compounds: glucose (GLU), ascorbic acid (AA), uric acid (UA), pyruvic acid (PA), dopamine (DOP) and glutamic acid (GA); and cotinine (COT) as the main metabolite of nicotine (NIC). These compounds were analyzed by adding them to the artificial sweat solution (pH 7.4) containing 20 μM in 1-fold

mass ratio of NIC (1:1), and the changes in the peak current of 20 μM NIC were compared in the presence of selected interfering compounds. It was considered that tested compounds strongly interfere with the determination of nicotine if they give signal changes more than 10%.

The selectivity of the method was investigated by testing of the most common interfering compounds by adding them to a solution containing NIC in same concentration (20 μM) (Fig.5).

Tested common ions did not interfere with NIC detection. Organic compounds such as AA, DOP and UA can be expected in the human body fluids. They normally appear in human blood in the concentration ranges of 34-80, <1.0, and 178-416 μM respectively and therefore the inventors have chosen these quantities as a reference for human sweat. In this study, only DOP caused remarkable changes on the peak current of NIC. Hence, this interfering compound does not limit the practical applicability of the sensor of the invention since the tested DOP concentration in this study was significantly higher than that usually expected in biological samples. Cotinine has not shown any characteristic effect on the current response of NIC. These results indicate good selectivity of the method toward the application in sweat determination.

***<u>Example 5: Sample analysis</u>***

At best of the inventor's knowledge, the electrochemical analysis of NIC in sweat samples has not been published in literature so far. Therefore, SPCEs were used in this study for sensing of NIC in artificial sweat and in human sweat collected with a sweat patch in order to explore the practical applicability and accuracy of the proposed method for the detection of NIC on the human skin. All samples were analyzed in triplicate, and results are given as mean value of three measurements.

Multiple additions of standard solution of nicotine in the concentrations 10, 100, 200 and 300 μM were made into the artificial sweat solution and the voltammetric responses were monitored using SPCE and DPV under optimized experimental conditions.

Fig.6 shows voltammograms by multiple additions of NIC standard solution to the artificial sweat sample at the concentrations of 10, 100, 200 and 300 μM. It is evident that the oxidation peak of NIC increases proportionally after each addition with a slightly shift to more positive potentials compared with PBS. The obtained recoveries were in the range of 91 - 102% (Table 1) showing a minor impact of the matrix and indicating satisfactory capability of the method for accurate and quick determinations of NIC in sweat samples.

Results regarding the applicability of sweat patches for the monitoring of nicotine in human sweat of a heavy and light smoker are shown in Table 1 and 2. The amount of NIC absorbed on the patch of the heavy smoker was higher than on the patch of the light smoker, corresponding to absolute amounts of NIC/ patch of 0.047 μmol (7.5 μg) and 0.009 μmol (1.4 μg) respectively. Spiking of extracts with different nicotine concentrations gave satisfactory recoveries (97 - 106%) showing a promising feasibility of sweat patches for sweat collection for the determination of nicotine in sweat samples.

**Table 1.** Determination of nicotine in artificial sweat solutions using the proposed method.

| Artificial Sweat | | |
|---|---|---|
| **Added (μM)** | **Found (μM)** | **Recovery (%)** |
| 0.0 | 0.0 | - |
| 10.0 | 10.1±0.2 | 102 |
| 100.0 | 90.5±1.4 | 91 |
| 200.0 | 186.6±1.7 | 93 |
| 300.0 | 276.0±2.7 | 92 |

**Table 2.** Determination of nicotine in human sweat as spiked in sweat patch extracts using the proposed method.

| Heavy Smoker | | | Light Smoker | | |
|---|---|---|---|---|---|
| **Added (μM)** | **Found (μM)** | **Recovery (%)** | **Added (μM)** | **Found (μM)** | **Recovery (%)** |
| - | 23.4 | - | - | 4.4 | - |
| 10.0 | 32.3±0.2 | 97 | 10.0 | 15.3±0.8 | 106 |
| 30.0 | 54.1±0.3 | 101 | 30.0 | 34.6±0.7 | 101 |
| 80.0 | 106.3±0.9 | 103 | 80.0 | 83.0±1.1 | 98 |

## Claims

1. An electrochemical method for detecting nicotine in sweat, in particular in human sweat, the method comprising the steps of:

   (a) providing a solution comprising or consisting of sweat, and
   (b) achieving an electrochemical measurement of the solution resulting from step a) with an electrochemical sensor.

2. The electrochemical method according to claim 1, **characterized in that** the electrochemical measurement is a voltammetric measurement, in particular a differential pulse voltammetric measurement.

3. The electrochemical method according to any one of the preceding claims, **characterized in that** the electrochemical sensor comprises a screen-printed carbon electrode.

4. The electrochemical method according to any one of the preceding claims, **characterized in that** step a) is carried out by collecting the sweat and diluting it in a buffered electrolytic solution, preferably having a pH equal to the physiological pH, typically between 5 and 8.

5. The electrochemical method according to any of the preceding claims, **characterized in that** the method can detect nicotine in a minimal concentration of at least 0.10 $\mu$M, preferably of at least 0.30 $\mu$M.

6. The electrochemical method according to any of the preceding claims, **characterized in that** nicotine is quantified in sweat.

7. The electrochemical method according to claim 6, **characterized in that** said method comprises a further step c) of quantifying nicotine in sweat by using the voltammogram resulting from step b), wherein said step c) is achieved by using a calibration curve of $i=f(E)$ made starting from artificial sweat samples comprising different concentrations of nicotine.

8. A device for electrochemical detection of nicotine in sweat, in particular in human sweat, said device comprising an electrochemical sensor.

9. The device according to claim 8, **characterized in that** the electrochemical sensor is suitable to be immersed or contacted in a solution comprising or consisting of sweat.

10. The device according to claim 8 or 9, **characterized in that** the electrochemical sensor is suitable to perform a voltammetric measurement, in particular a differential pulse voltammetric measurement.

11. The device according to any one of claims 8 to 10, **characterized in that** the electrochemical sensor comprises a screen-printed carbon electrode.

12. The device according to any one of claims 8 to 11, **characterized in that** the device further quantifies nicotine in sweat.

13. The device according to any one of claims 8 to 12, said device being included in a dermal patch, preferably a self-adhesive dermal patch.

14. Use of the device according to any one of claims 8 to 13 for electrochemical detection of nicotine in sweat.

15. A dermal patch comprising a device for electrochemical detection of nicotine in sweat as defined in any of claims 8 to 13.

Figure 1

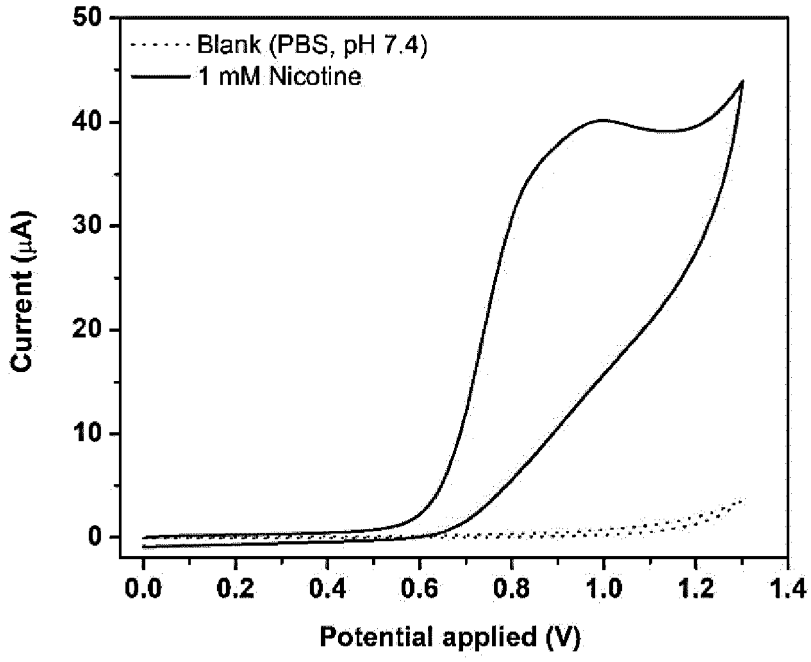


Figure 2

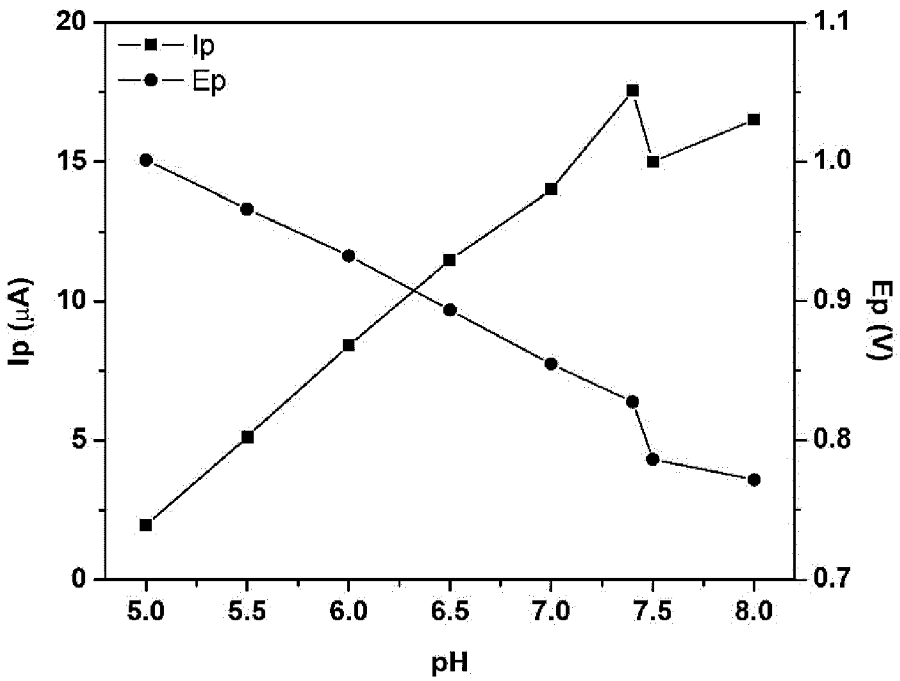

Figure 3

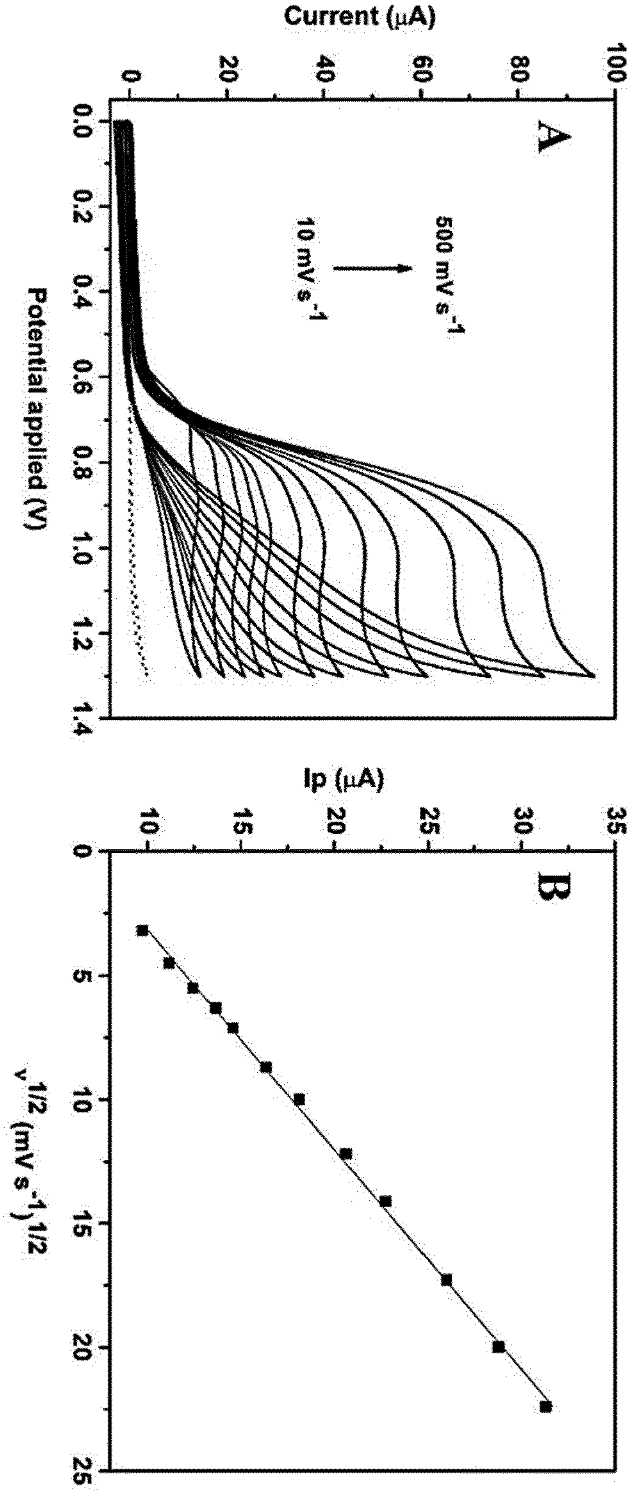
A
Current (μA)
0
20
40
60
80
100
Potential applied (V)
0.0
0.2
0.4
0.6
0.8
1.0
1.2
1.4
500 mV s-1
10 mV s-1
B
Ip (μA)
10
15
20
25
30
35
v1/2 (mV s-1)1/2
0
5
10
15
20
25

## Figure 4

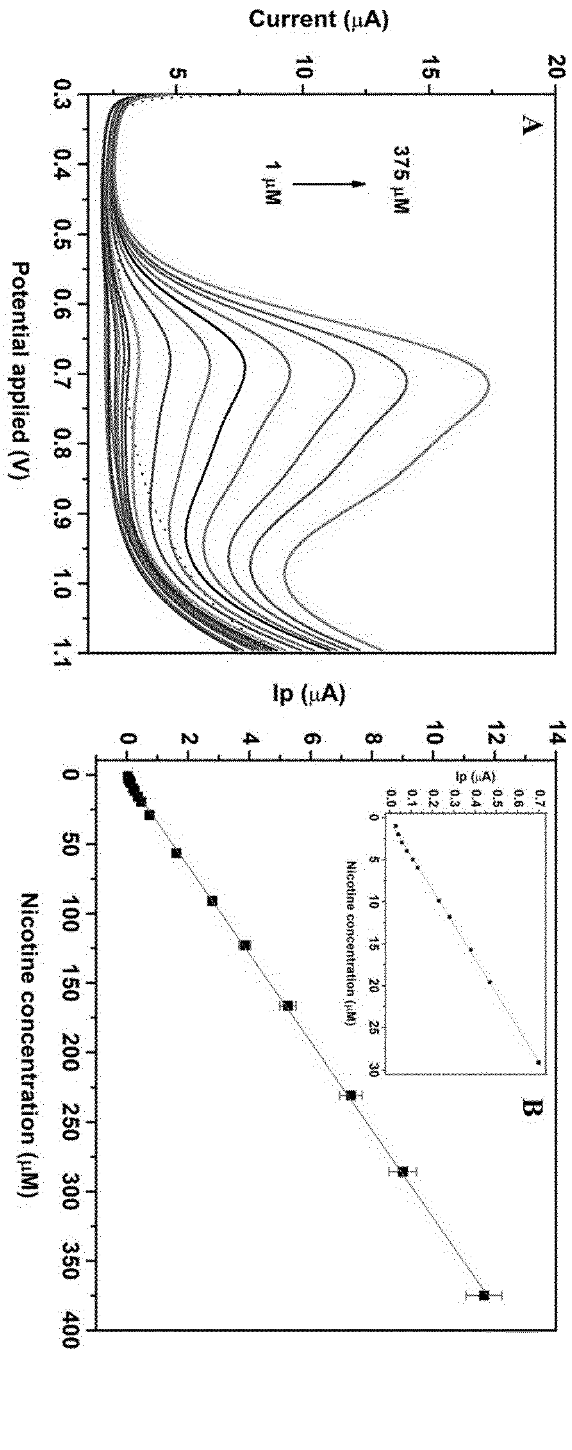
A
Current (µA)
Potential applied (V)
375 µM
1 µM
B
Ip (µA)
Nicotine concentration (µM)

Figure 5

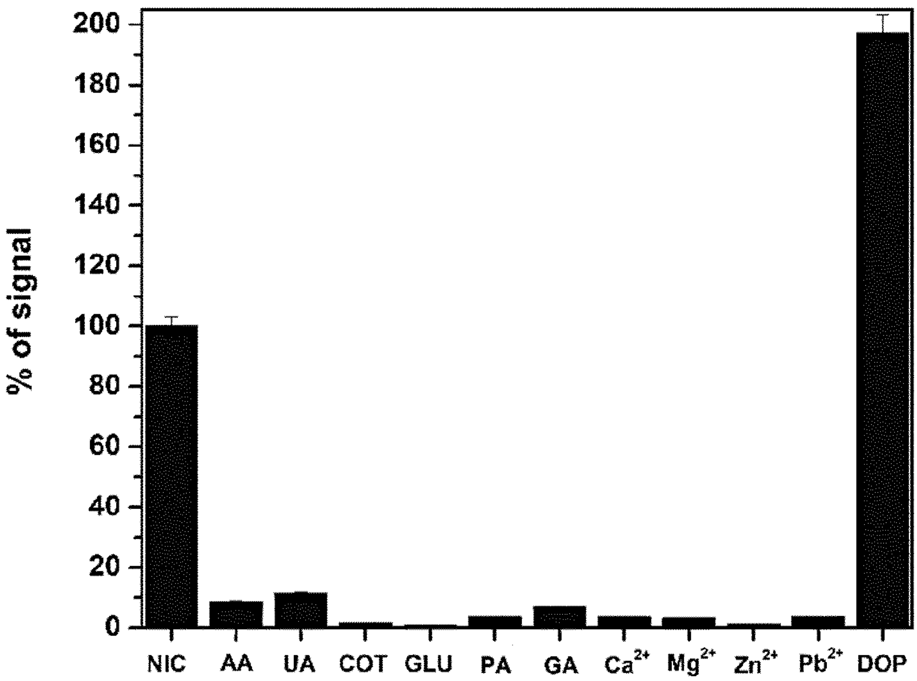


Figure 6

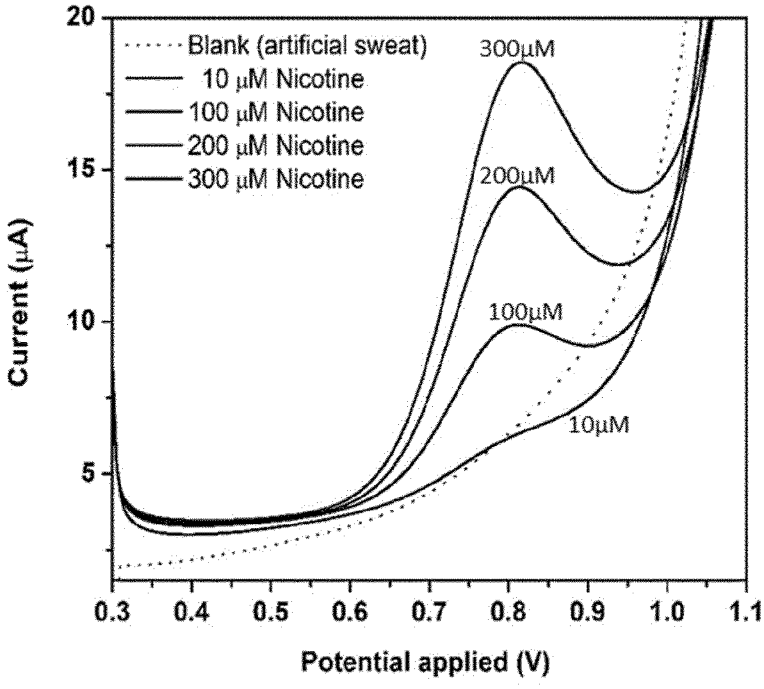

Europäisches Patentamt
European Patent Office
Office européen des brevets

# EUROPEAN SEARCH REPORT

Application Number
EP 19 30 5595

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | SHUANG LI ET AL: "Fingerprints mapping and biochemical sensing on smartphone by electrochemiluminescence", SENSORS AND ACTUATORS B: CHEMICAL, vol. 285, 8 January 2019 (2019-01-08), pages 34-41, XP055630588, NL ISSN: 0925-4005, DOI: 10.1016/j.snb.2019.01.035 * the whole document * * in particular: * * abstract * * figure 1(b)3(a) * * section 2.4 * * section 3.1 * * section 3.2 * ----- | 1-15 | INV. A61B5/145 A61B5/1477 A61B5/00 G01N27/42 |
| X | US 2018/271423 A1 (AGARWAL ANAND K [US] ET AL) 27 September 2018 (2018-09-27) * paragraphs [0059], [0062], [0092], [0104] * ----- | 1-15 | |
| | | | TECHNICAL FIELDS SEARCHED (IPC) |
| A | WANG S J ET AL: "Low potential detection of nicotine at multiwalled carbon nanotube-alumina-coated silica nanocomposite", ELECTROCHEMISTRY COMMUNICATIONS, ELSEVIER, AMSTERDAM, NL, vol. 11, no. 4, 1 April 2009 (2009-04-01), pages 733-735, XP026075542, ISSN: 1388-2481, DOI: 10.1016/J.ELECOM.2009.01.026 [retrieved on 2009-01-29] * abstract * ----- | 4 | A61B G01N |
| A | US 2018/263539 A1 (JAVEY ALI [US] ET AL) 20 September 2018 (2018-09-20) * paragraph [0097] * ----- | 7 | |

-/--

The present search report has been drawn up for all claims

1

| Place of search | Date of completion of the search | Examiner |
|---|---|---|
| The Hague | 10 October 2019 | Albrecht, Ronald |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 19 30 5595

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | KINTZ P ET AL: "Nicotine monitoring in sweat with a sweat patch", JOURNAL OF CHROMATOGRAPHY B: BIOMEDICAL SCIENCES & APPLICATIONS, ELSEVIER, AMSTERDAM, NL, vol. 705, no. 2, 13 February 1998 (1998-02-13), pages 357-361, XP004108730, ISSN: 1570-0232, DOI: 10.1016/S0378-4347(97)00551-3 * abstract * ----- | 13,15 | |

TECHNICAL FIELDS SEARCHED (IPC)

1

The present search report has been drawn up for all claims

| Place of search | Date of completion of the search | Examiner |
|---|---|---|
| The Hague | 10 October 2019 | Albrecht, Ronald |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 19 30 5595

This annex lists the patent family members relating to the patent documents cited in the above-mentioned European search report. The members are as contained in the European Patent Office EDP file on
The European Patent Office is in no way liable for these particulars which are merely given for the purpose of information.

10-10-2019

| Patent document cited in search report | Publication date | Patent family member(s) | Publication date |
|---|---|---|---|
| US 2018271423 A1 | 27-09-2018 | US 2018271423 A1<br>WO 2017062591 A1 | 27-09-2018<br>13-04-2017 |
| US 2018263539 A1 | 20-09-2018 | US 2018263539 A1<br>WO 2017058806 A1 | 20-09-2018<br>06-04-2017 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **KARACONJI, I.B.** *Arh. Hig. Rada Toksikol,* 2005, vol. 56, 363-371
- **ŠVORC L. et al.** *Diam. Relat. Mater.,* 2014, vol. 42, 1-7
- **HIGHTON, L. et al.** *Electroanalysis,* 2009, vol. 21, 2837
- **DOLLERY et al.** *Br. Med. J.,* 1975, vol. 4, 313-316
- **KINTZ et al.** *J. Chromatogr. B Biomed. Appl.,* 1998, vol. 705, 357-361